(19) Europäisches Patentamt
European Patent Office
Office européen des brevets

(11) Publication number: **0 174 395 B1**

(12) **EUROPEAN PATENT SPECIFICATION**

(45) Date of publication of patent specification: **24.05.89**

(51) Int. Cl.$^4$: **C 07 H 17/08**, A 61 K 31/70

(21) Application number: **84116047.6**

(22) Date of filing: **01.02.82**

(60) Publication number of the earlier application in accordance with Art. 76 EPC: **0 057 489**

(54) **Salts of erythromycin with mercapto-succinic acid having therapeutic activity, process for their preparation and pharmaceutical compositions containing them.**

(30) Priority: **02.02.81 FR 8101930**

(43) Date of publication of application: **19.03.86 Bulletin 86/12**

(45) Publication of the grant of the patent: **24.05.89 Bulletin 89/21**

(84) Designated Contracting States: **AT BE CH DE GB LI LU NL SE**

(56) References cited:
**US-A-3 847 896**

**CHEMICAL ABSTRACTS, vol. 70, no. 13, 31st March 1969, page 209, no. 56120b, Columbus, Ohio, US; I.N. BEL'GOVA: "Action of mercapto compounds on novembichin poisoning in mice of different ages", & FARMAKOL. TOKSIKOL. 1968, 31(6), 738-41**

**CHEMICAL ABSTRACTS, vol. 72, no. 15, 13th April 1970, page 125, no. 76027w, Columbus, Ohio, US; K. TANAKA et al.: "Mode of action of siomycin", & J. ANTIBIOT. 1970, 23(1), 13-19**

(73) Proprietor: **Pierrel S.p.A.
Via Depretis, 88
I-80100 Napoli (IT)**

(72) Inventor: **Gonella, Jacques
Bahnhofstrasse 29
Zollikom Zürich (CH)**

(74) Representative: **Dragotti, Gianfranco
SAIC BREVETTI s.a.s. Viale Bianca Maria, 15
I-20122 Milano (IT)**

**EP 0 174 395 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to novel thiolic derivatives of erythromycin and of the propionic ester of erythromycin, namely salts thereof with mercapto-succinic acid.

The exploitation of the therapeutical properties of thiolic compounds in combination with the properties of antibiotics has been already attempted.

However (C.A. *93*, 38297 f) it was found that acetylcysteine and the derivatives thereof have an inhibiting action with respect to the activity of antibiotics.

US—A—384789 relates to the thiolic esters of derivatives of erythromycin with bicarboxylic acids, particularly succinic and glutaric acids, having improved taste with respect to erythromycin and a favourable absorption, when orally administered.

It has been now suprisingly found that the compounds of the present invention find therapeutical use in the case in which erythromycin or the propionic ester thereof are already used and are generally characterized by a very low toxicity and by a high hematic concentration; these aspects are very important from the therapeutical point of view.

The derivatives according to the present invention have the following general formula:

$$R—X \qquad (I)$$

wherein R represents the radical of mercapto-succinic acid or of the sodium salt thereof having the formula:

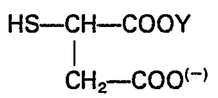

Y being H or Na and

X is the radical of erythromycin or of the propionic ester of erythromycin having the formula:

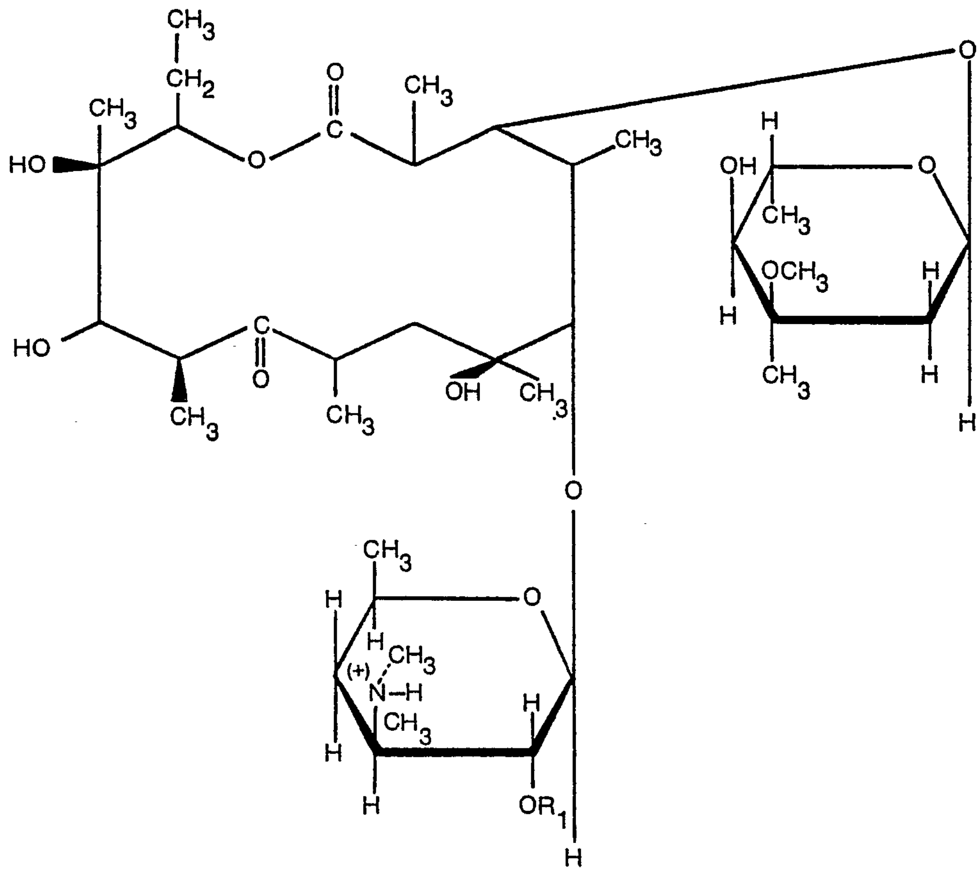

in which $R_1$ is H or $CH_3—CH_2—CO$.

The compounds according to the invention are white, microcrystalline powders.

Generally, the salt containing erythromycin monopropionyl ester is less soluble than the corresponding salt of erythromycin base; for both derivatives, moreover, the solubility increases in solvent mixtures, such as water-ethanol (1—5%) and water ethylene glycol (1—5%).

Their use is anyhow foreseen under all pharmaceutical forms, capsules, solutions, injectable preparations, aerosols, creams, powders and suspensions, both for human being and for veterinary use.

Particularly for the salt of erythromycin base there are foreseen pharmaceutical preparations of the injectable type or administerable by aerosol, whereas for the derivatives of propionyl erythromycin there are preferred the orally administerable formulations and the suspensions. The compounds are affected by sun light, humidity and heat, and are true salts, both from the chemical and from the physical point of view.

The method for the preparation of the erythromycin or of propionylester of erythromycin according to the present invention comprises reacting erythromycin base or the propionic ester of erythromycin with mercapto succinic acid or sodium mercapto hemisuccinate, in a stoichiometrical ratio or in the presence of a slight excess of the antibiotic nucleus, and is characterized in that the reaction is carried out in an organic solvent, at a temperature of between 20 and 40°C and in the presence of water in an amount not greater than 20%.

In fact, it has been found, in a suprising manner, that the presence of water preferably in an amount of 4—5% by volume with respect to the reaction solvent permits the reaction to be completed with exceedingly good results. On the contrary, water amounts greater than 20% may cause the reaction product to be dissolved again in the aqueous/organic mixture. The following examples, only given for illustrative purpose, disclose the preparation of the derivatives of the invention.

Example 1

Erythromycin salt with mercapto-succinic acid

46.26 g (0.063 moles) of dry erythromycin base (namely a 2% excess with respect to the purely stechiometrical reaction ratio) are dissolved in 350 mls of acetone at a temperature of between 15 and 40°C. This solution is added with 4.64 g of mercaptosuccinic acid, to obtain a clear and colorless solution.

Then 15 mls of water are added dropwise, a salt is very rapidly formed, which precipitates and tends to prevent the stirring. There are formed white microcrystals and the stirring is continued for about 1 hour.

The product is filtered under vacuum by means of a Buchner Funnel and acetonic mother liquors are obtained, being slightly yellow straw-coloured, which after evaporation give negligable amounts of crystals.

The product is dried at a maximum temperature of 40°C and 48.5 g of a crystalline product are thus obtained (molecular weight 1618) with a yield of 97%.

The melting point is 133—140°C (with decomposition).

Fig. 1 shows the IR spectrum.

Example 2

Salts of propionylerythromycin with sodium alpha-mercapto-hemisuccinate

3.8 g of alpha-mercapto-succinic acid are dissolved in 10 mls of water with 2.12 g of sodium bicarbonate until the effervescence is terminated.

20 g of erythromycin propionate are dissolved at 30°C in 20 mls of acetone and 100 mls of methylisobutylketone.

Under stirring, the solution of erythromycin propionate is added with the solution of sodium alpha-mercaptohemisuccinate and the mixture is heated to a maximum temperature of 40°C, the stirring being continued.

The salt is slowly formed and precipitates upon cooling.

The mixture is cold filtered under reduced pressure and washed with little methylisobutylketone.

The yield is 18.5 g (76.5% of the theoretical yield).

Fig. 2 shows the IR spectrum.

Example 3

Salt of erythromycin propionate with mercapto-succinic acid

100 g of erythromycin propionate are dispersed in 400 mls of acetone, at room temperature and under stirring. Then 9.5 g of mercapto-succinic acid are added, by maintaining the mixture under stirring until a totally clear solution is obtained.

Then 20 mls of water are slowly added.

The salt precipitates with a yield of 96%, about 1 hour after the water addition.

The melting point is 122—127°C.

The compounds of the invention have been subjected to toxicological, pharmacological and pharmacodynamic tests aiming to assess the toxicity (and consequently the possibility of use in the therapeutical field) and the therapeutical properties.

In the hereinafter stated results the compounds of the invention are indicated by the following abbreviations:

RV/01-erythromycin alpha-mercaptosuccinate;

RV/11-erythromycin-monopropionate-alpha-mercaptosuccinate;

A) *Acute toxicity*

The acute toxicity was assessed in Swiss white mice by oral and intravenous route. The $LD_{50}$ values have been calculated by the methods of Probits, starting from the mortality recorded 10 days after the treatment.

| Compound | Administration route | $LD_{50}$ mg.kg (reliability limits) |
|---|---|---|
| erythromycin | os | >3000 |
| erythromycin hydrochloride | i.v. | 376.58 (478.78—296.20) |
| RV 01 | os | >3000 |
| RV 01 | i.v. | 458.49 (518.06—405.77) |
| RV 11 | os | >3000 |

B) *Antibacterial activity in vitro*

The antibacterial activity in vitro of the compounds of the invention was assessed by comparing the different minimum inhibiting concentrations (MIC) with those of erythromycin base and of erythromycin estolate in Gram-positive and Gram-negative strains cultivated on Brain Heart Infusion (Difco). The tested samples of erythromycin estolate and RV 01, were previously hydrolized according to the prescription given in U.S. Pharmacopoeia, XX edition, page 1347.

In the following table I the MIC/ml values are indicated corresponding to the lowest concentration capable of inducing a complete inhibition of the bacterial development.

| Strain | | Erythromycin | Erythromycin Estolate | RV 01 | RV 11 |
|---|---|---|---|---|---|
| 1) Staph.albus | SS 03 | 0,05 | 0,05 | 0,1 | 0,05 |
| 2) | SS 04 | 0,05 | 0,05 | 0,05 | 0,05 |
| 3) Staph.aureus | SS 07 | 0,025 | 0,205 | 0,05 | 0,05 |
| 4) | SS 08 | 0,025 | 0,025 | 0,025 | 0,025 |
| 5) | SS 09 | 0,1 | 0,05 | 0,1 | 0,1 |
| 6) | SS 10 | 0,1 | 0,2 | 0,05 | 0,025 |
| 7) Strept. haemolyticus | SS 17 | 3 | 3 | 3 | 1,5 |
| 8) | SS 18 | 1,5 | 3 | 1,5 | 1,5 |
| 9) Strept.faecalis | SS 19 | 0,1 | 0,1 | 0,1 | 0,05 |
| 10) S.lutea ATCC 9341 | | 0,01 | 0,01 | 0,01 | 0,01 |
| 11) B.subtilis ATCC 6633 | | 0,025 | 0,01 | 0,01 | 0,025 |
| 12) | SS 127 | 0,05 | 0,025 | 0,01 | 0,05 |
| 13) B.cereus ATCC 11778 | | 0,4 | 0,8 | 0,4 | 0,8 |
| 14) B.anthranis | SS 08 } | 0,4 | 0,4 | 0,4 | 0,8 |
| 14bis) | SS 09 } | 0,4 | 0,4 | 0,4 | 0,4 |
| 15) Br.melitensis | SS 19 | 3 | 3 | 3 | 3 |
| 16) Br.abortus | SS 7 | 12 | 6 | 6 | 6 |
| 17) Citrobacter | SS 05 } | >200 | >200 | >200 | >200 |
| 18) | SS 09 } | >200 | >200 | >200 | >200 |

| Strain | | Erythromycin | Erythromycin Estolate | RV 01 | RV 11 |
|---|---|---|---|---|---|
| 19) S.infantis | SS 08 | 100 | 100 | 100 | 100 |
| 20) S.heidelberg | SS 18 | 100 | 100 | 100 | 100 |
| 21) E.cloacae | SS 07 | >200 | >200 | >200 | >200 |
| 22) E.acrogenes | SS 011 | 100 | 50 | 50 | 100 |
| 23) Ps.aeruginosas | SS 10 | 200 | 100 | 200 | 100 |
| 24) | SS 12 | >200 | 200 | 200 | 200 |
| 25) E.coli | SS 04 | 100 | 100 | 50 | 50 |
| 26) | SS 05 | 50 | 100 | 50 | 100 |
| 27) | SS 06 | 100 | 50 | 100 | 50 |
| 28) | SS 07 | 50 | 50 | 25 | 50 |
| 29) Pr.mirabilis | SS 09 | >200 | >200 | >200 | >200 |
| 30) Pr.vulgaris | SS 10 | >200 | >200 | >200 | >200 |
| 31) Kl.aerogenes | SS 71 | 50 | 100 | 100 | 100 |
| 32) Kl.pneumoniae | SS 04 | 100 | 100 | 100 | 200 |
| 33) Sr.marcescens | SS 03 | 200 | >200 | 200 | 200 |
| 34) C.diphtheriae | SS 19 | 0,01 | 0,025 | 0,025 | 0,01 |
| 35) D.pneumoniae | SS 23 | 0,05 | 0,05 | 0,05 | 0,025 |
| 36) N.gonorreae | SS 27 | 0,8 | 0,8 | 0,8 | 0,4 |
| 37) N.meningitidis | SS 04 | 0,8 | 1,5 | 0,8 | 1,5 |
| 38) H.influentiae | SS 02 | 1,5 | 1,5 | 1,5 | 0,8 |
| 39) H.pertussia | SS 03 | 0,4 | 0,4 | 0,4 | 0,4 |
| 40) H.tubercolosis | SS 03 | 50 | 50 | 50 | 50 |
| 41) C.albicans | SS 04 | 100 | 200 | >200 | 200 |
| 42) Cl.tetani | SS 17 | 0,4 | 0,2 | 0,2 | 0,2 |

Swiss white mice of 20 g body weight were used, infected by parenteral administration of lethal amounts of pathogenic genes of Staph. aureus SS 07 and D. pneumoniae SS 23. The protecting activity expressed ad $ED_{50}$ mg/kg was assessed for erythromycin base, erythromycin estolate, RV 01, and RV 11 administered by oral route as well as for erythromycin ethyl succinate and RV 01 administered by subcutaneous route.

The antibiotics were administered at the time of injecting the infecting germ and 6 and 24 hours later. On the basis of the survival after 7 days, the $ED_{50}$ values were determined, as reported in the following tables II and III, wherein the reliability limits are indicated in brackets.

TABLE II

Experimental Infection by Staph. aureus SS 07 ($ED_{50}$ mg/kg of Erythromycin)

| Compound | Administration route | $ED_{50}$ mg/kg (reliability limits) |
|---|---|---|
| erythromycin ethyl succinate | s.c. | 1.03 (1.34—0.79) |
| RV 01 | s.c. | 0.95 (1.28—0.71) |
| erythromycin | os | 2.09 (2.36—1.86) |
| erythromycin estolate | os | 2.20 (2.44—1.99) |
| RV 01 | os | 2.36 (2.68—2.08) |
| RV 11 | os | 2.14 (2.40—1.91) |

TABLE III

Experimental Infection by D. pneumoniaes SS 23 ($ED_{50}$ mg/kg of Erythromycin)

| Compound | Administration route | $ED_{50}$ mg/kg (reliability limits) |
|---|---|---|
| erythromycin ethyl succinate | s.c. | 54.23 (69.76—42.16) |
| RV 01 | s.c. | 52.70 (64.91—42.79) |
| erythromycin | os | 113.53 (147.33—87.48) |
| erythromycin estolate | os | 127.89 (156.42—104.57) |
| RV 01 | os | 108.98 (137.60—86.32) |
| RV 11 | os | 100.41 (122.52—82.30) |

D) *Pharmacodynamics*

a) In the rat

The absorption by oral route of the salts of erythromycin was investigated in Sprague-Dawley male rats which were administered, in group of 6 animals fasted since 12 hours, with erythromycin, erythromycin estolate, RV 01 and RV 11 at the dose of 100 mk/kg (expressed as erythromycin) by oral route (gastric probing).

The blood samples were taken 0.30, 1, 2, 3, 4, 5 and 6 hours after the administration.

For the dosing the microbiological method, and as the test micro-organism, B subtilis were used. The experimental results are reported in the following table IV:

TABLE IV

Hematic Levels in the Rat (Average Plus Standard Error)

| Compound | N. of animals | μg/ml after . . . hours 0.30 | 1 | 2 | 3 | 4 | 5 | 6 |
|---|---|---|---|---|---|---|---|---|
| erythromycin | 6 | 0.71<br>0.19 | 1.03<br>0.23 | 1.15<br>0.24 | 1.05<br>0.22 | 0.37<br>0.10 | 0.26<br>0.08 | 0.03<br>— |
| erythromycin estolate | 6 | 0.68<br>0.07 | 1.74<br>0.14 | 2.49<br>0.35 | 1.49<br>0.22 | 0.61<br>0.10 | 0.37<br>0.05 | 0.04<br>0.01 |
| RV 01 | 6 | 0.48<br>0.11 | 0.79<br>0.15 | 1.18<br>0.24 | 0.77<br>0.17 | 0.44<br>0.12 | 0.24<br>0.08 | 0.03<br>0.05 |
| RV 11 | 6 | 0.72<br>0.16 | 1.64<br>0.19 | 2.50<br>0.27 | 1.31<br>0.21 | 0.58<br>0.22 | 0.38<br>0.14 | 0.08<br>0.02 |

b) In the healthy volunteer

Group of 5 healthy volunteers, fasted since 24 hours, were administered by oral route with erythromycin estolate and RV 11 at a dose of 500 mg of erythromycin.

The blood samples were taken 0.30, 1, 2, 3 and 4 hours after the administration.

For the dosing, the microbiological method using a B. subtilis strain was adopted.

The results are indicated in table V.

TABLE V

Hematic Levels in the Healthy Volunteer (Average Plus Standard Error)

| Compound | N. of animals | mcg/ml after . . . hours 0.30 | 1 | 2 | 3 | 4 | 5 | 6 |
|---|---|---|---|---|---|---|---|---|
| erythromycin estolate | 5 | 0.27 | 0.58 | 2.10 | 1.41 | 1.31 | — | — |
| | | 0.05 | 0.09 | 0.17 | 0.07 | 0.08 | — | — |
| RV 11 | 5 | 1.28 | 1.70 | 2.16 | 1.48 | 1.21 | — | — |
| | 5 | 0.12 | 0.09 | 0.12 | 0.08 | 0.05 | — | — |

E) *Mucolytic activity*

There was used the method described by Quevauviler et al (Thérapie 22, 485, 1967) according to which a bronchial hypersecretion is induced after exposure of the animal to an aerosol treatment with $SO_2$. The test was carried out on Sprague Dawley male rats. All rats were daily subjected to $SO_2$ inhalations, at a 0.03% concentration. After 50 hours of inhalation, the animals were divided in groups comprising 10 animals each.

One group was not treated (control animals), whereas the other animals were treated, two hours per day, for 15 days consecutively with inhalations of $SO_2$ and with erythromycin estolate and RV 11, by oral route at the dose of 500 mg/kg as well as with erythromycin ethyl succinate and RV 01, by intramuscolar route at the dose of 250 mg/kg.

The day after the last treatment, the anesthetized animals were sacrificed, and the lungs were removed and prepared for the microscopical and macroscopical examination. The results are indicated in the following Table VI.

TABLE VI

Qualitative and Quantitative Evaluation of the Obstruction of the Bronchial Tract (% Incidence)

| Treatment | administration route | Total obstruction compact plug | nodular mass | pimply mass | Partial obstruction |
|---|---|---|---|---|---|
| $SO_2$ (control) | — | 40 | 13.33 | 6.66 | 26.66 |
| $SO_2$ + erythromycin estolate 500 mg/kg | oral | 46.66 | 20 | 6.66 | 20 |
| $SO_2$ + RV 11 500 mg/kg | oral | 20 | 6.66 | 6.66 | 13.33 |
| $SO_2$ + RV 01 250 mg/kg | i.m. | 26.66 | 6.66 | 13.33 | 13.33 |

Thereafter the histological examination of both control and treated animals was carried out.

1) *Control animals subjected to a $SO_2$ inhalation*

The macroscopical bronchial obstructions as detected by the hystological examination correspond to a mucus mass admixed with fibrin and infiltrated with polynuclear elements. The hypersecretion does equally concern the peripheral bronchioles and the alveoles.

As regards the bronchial epithelium there is revealed a proliferation of cup-shaped cells. The hyperplasy of the main bronchus appears as a stratification of 7 to 8 layers, associated to a bronchial hypertrophy.

2) *Animals treated with erythromycin estolate at the dose of 500 mg/kg by oral route and with erythromycin ethyl succinate at the dose of 250 mg/kg by intramuscular route*

From the histological examination it appears that the bronchial obstruction is formed by abundant mucus admixed with fibrin.

Due to the irritating effect of $SO_2$, the bronchial epithelium reacts through a proliferation of cells, mainly cup-shaped cells. The hyperplasy is associated in a number of cases to a bronchial hypertrophy. In some cases, mucus secreting cells appear in the peripheral bronchioles.

3) *Animals treated with RV 11, at the dose of 500 mg/kg, by oral route*

In the animals having, under macroscopical examination, a free non obstructed bronchial tract, the histological examination revealed the presence of bronchi having almost normal appearance. An epithelial hyperplasy with a number of cup-shaped cells and reduced intra-bronchial muco-purulents masses was detected.

The hystological appearance of the obstructed bronchial tracts is fully like that of the control animals.

4) *Animals treated with RV 01, at the dose of 250 mg/kg, by intramuscular route*

The histological examination as carried out in the animals which under macroscopical examination had free non obstructed bronchi, revealed a practically normal appearance. In some animals, the presence of an epithelial hyperplasy was detected with cup-shaped cells associated to intra-bronchial muco-purulent masses.

The histological examination of the animals showing an obstructed bronchial tract, revealed an appearance like that of the control animals.

*Conclusions*

From the toxicity tests it results that the erythromycin salts are devoid of toxicity when administerd by oral route. When injected, they can be slightly toxic and have however $LD_5$ values comparable with that of erythromycin.

From the tests of antibacterial activity in vitro it results that the erythromycin salts are active in a proportion like that of the erythromycin base.

From the tests of antibacterial activity in vivo and from the pharmacodynamic tests it results that the compound RV 01 is poorly absorbed by oral route in and a rate like that of erythromycin base.

The propionic derivative RV 11, is absorbed by oral route in a rate practically analogous to that of erythromycin estolate. Contrarywise to erythromycin estolate, the drugs according to the invention are endowed with a good mucolytic activity which is revealed in the animals treated by $SO_2$ inhalation.

**Claims**

1. Thiolic derivatives of erythromycin and of propionic ester of erythromycin having the formula:

$$R{-}X \qquad (I)$$

wherein

R represents the radical of mercapto-succinic acid or of the sodium salt having the formula:

$$\begin{array}{l} HS{-}CH{-}COOY \\ \quad\;\;\; | \\ \quad\;\; CH_2{-}COO^{(-)} \end{array}$$

Y being H or Na and

X is the radical of erythromycin or of the propionic ester of erythromycin having the formula:

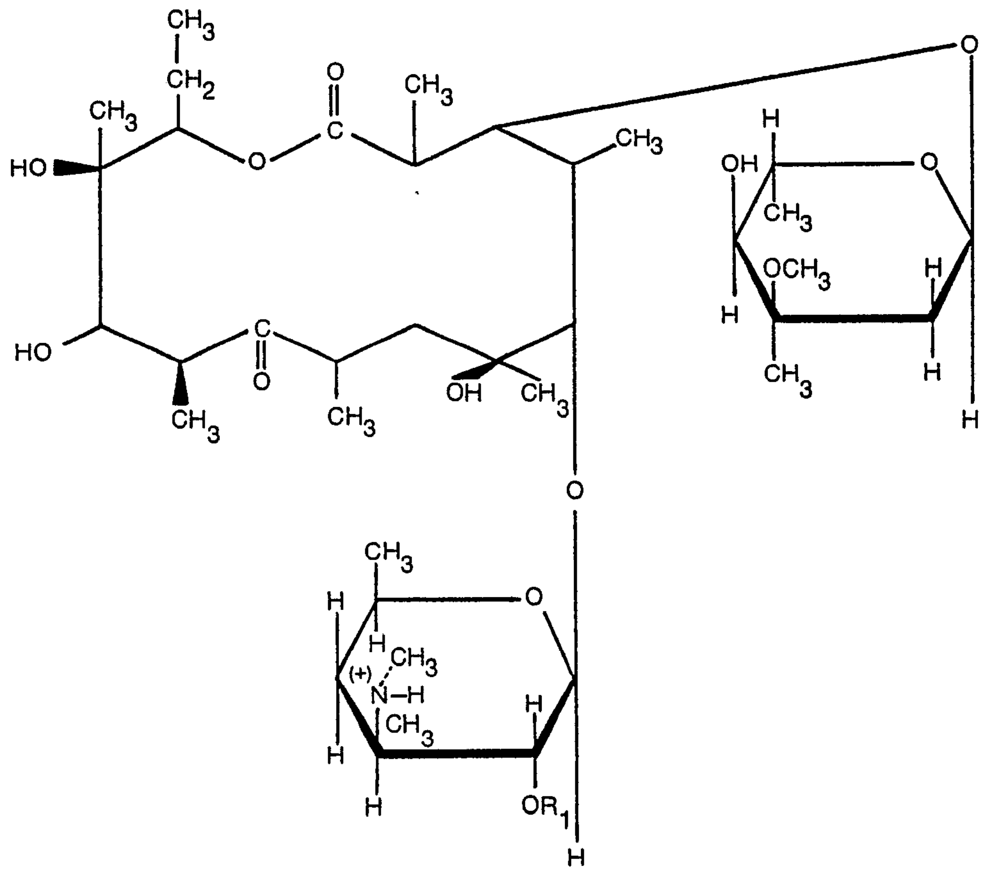

in which $R_1$ is H or $CH_3—CH_2—CO$.

2. Erythromycin salt with mercapto-succinic acid according to claim 1.

3. Salt of the propionic ester of erythromycin with mercapto-succinic acid according to claim 1.

4. Salt of the propionic ester of erythromycin with sodium alpha-mercapto hemisuccinate according to claim 1.

5. Salt of erythromycin with sodium alpha-mercapto hemisuccinate according to claim 1.

6. A process for the preparation of thiolic derivatives of erythromycin and of the propionic ester of erythromycin wherein erythromycin base or the propionic ester thereof is reacted with mercapto-succinic acid or the sodium mercapto hemisuccinate, characterized in that the reaction is carried out in an organic solvent, at a temperature of 20 to 40°C and in the presence of water.

7. A process according to claim 6, characterized in that the water present in the reaction solvent is not more than 20% by volume of the reaction solvent.

8. A process according to claim 7, characterized in that said water volume is 4 to 5% of that of the reaction solvent.

9. A process according to claim 6, characterized in that said organic solvent is acetone or methylisobutylketone.

10. Pharmaceutical preparation, characterized by containing, as the active ingredient, a thiolic derivative according to claim 1, as well as vehicles and fillers of usual type and acceptable from the pharmaceutical point of view.

11. Pharmaceutical preparation having antibacterial and mucolytic activity, characterized by containing, as the active ingredient, a derivative according to claim 1.

12. Pharmaceutical preparation according to claims 10 and 11, characterized by being in a form permitting the oral administration, particularly in form of capsules and suspensions, and by containing as the active ingredient an erythromycin derivative according to claim 1, as well as the usual excipients and vehicles.

13. Pharmaceutical preparation according to claims 10 and 11, characterized by being in form permitting the administration by parenteral route and by aerosol, and by containing as the active ingredient a derivative of the propionic ester of erythromycin according to claim 1, as well as the usual excipients and vehicles.

**Patentansprüche**

1. Thioldetivate von Erythromycin und von dem Propionester von Erytromycin der Formel:

$$R—X \qquad (I)$$

worin

R das Radikal der Mercaptobernsteinsäure oder des Natriumsalsez davon der Formel

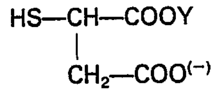

bedeutet, worin

Y H oder Na bedeutet und

X das Radikal von Erythromycin oder von dem Propionester von Erythromycin der Formel

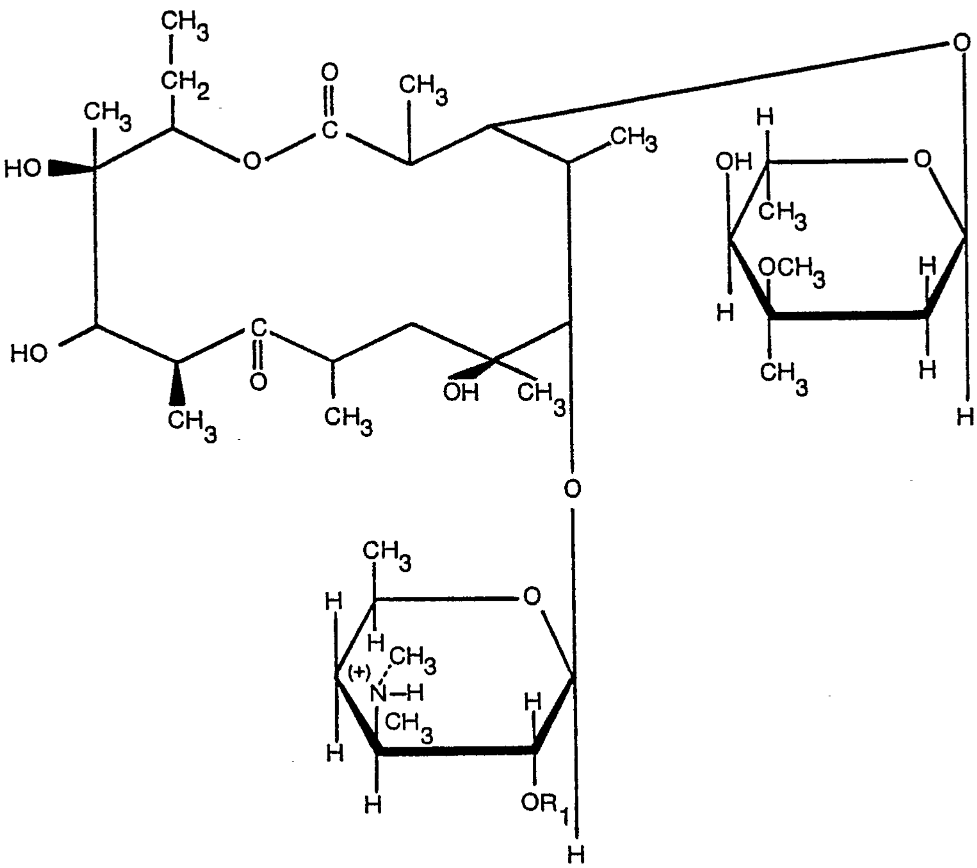

bedeutet, worin $R_1$ H oder $CH_3—CH_2—CO$ bedeutet.

2. Erytromycinsaltz mit Mercaptobernsteinsäure nach Anspruch 1.

3. Salz des Propionesters von Erythromycin mit Mercaptobernsteinäure nach Anspruch 1.

4. Salz des Propionesters von Erythromycin mit Natrium-α-mercaptohemisuccinat nach Anspruch 1.

5. Salz von Erythromycin mit Natrium-α-mercaptohemisuccinat nach Anspruch 1.

6. Verfahren zur Herstellung von Thiolderivaten von Erythromycin und von dem Propionester von Erythromycin, worin die Erythromycinbase oder der Propionester davon mit Mercaptobernsteinsäure oder dem Natriummercaptohemisuccinat umgesetzt wird, dadurch gekennzeichnet, daß die Reaktion in einem organischem Lösungsmitel bei einer Temperatur von 20 bis 40°C und in Gegenwart von Wasser durchgeführt wird.

7. Verfahren nach Anspruch 6, dadurch gekennzeichnet, daß das in dem Reaktionlösungsmittel vorhandene Wasser nicht mehr als 20 Vol.-% des Reaktionslösungsmittels darstellt.

8. Verfahren nach Anspruch 7, dadurch gekennzeichnet, daß das Wasservolumen 4 bis 5% der Reaktionslösung beträgt.

9. Verfahren nach Anspruch 6, dadurch gekennzeichnet, daß das organische Lösunsmittel Aceton oder Methylisobutylketon ist.

10. Pharmazeutische Zubereitung, dadurch gekennzeichnet, daß sie als Wirkstoff ein Thiolderivat nach Anspruch 1 enthalt ebenso wie übliche Träger- und Füllstoffe und welche aus pharmazeutischer verträglich sind.

11. Pharmazeutische Zubereitung mit antibakterieller und mukolytischer Wirksumkeit, dadurch gekennzeichnet, daß sie als Wirkstoff ein Derivat nach Anspruch 1 enthält.

12. Pharmazeutische Zubereitung nach Anspruch 10 und 11, dadurch gekennzeichnet, daß sie einer für orale Verabreichung geeigneten Form, insbesondere in Form von Kapseln und Sospensionen, vorliegt und daß sie als Wirkstoff ein Erythromycinderivat nach Anspruch 1 ebenso wie die üblichen Exzipinten und Träger enthält.

13. Pharmazeutische Zubereitung nach Anspruch 10 und 11, dadurch gekennzeichnet, daß sie in einer für parenterale Verabreichung und für die Verabreichung als Aerosol geeigneten Form vorliegt, und daß sie als wirkstoff ein Derivat des Propionesters von Erythromycin nach Anspruch 1 ebenso wie die üblichen Träger unf Exzipienten enthält.

**Revendications**

1. Dérivés thioliques d'érythromycine et d'ester propionique d'érythromycine répondant à la formule:

$$R—X \qquad (I)$$

dans laquelle R représente le radical d'acide mercapto-succinique ou de son sel de sodium répondant à la formule:

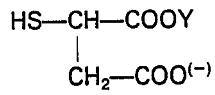

Y représentant H ou Na, et

X représente le radical d'érythromycine ou de l'ester propionique d'érythromycine répondant à la formule:

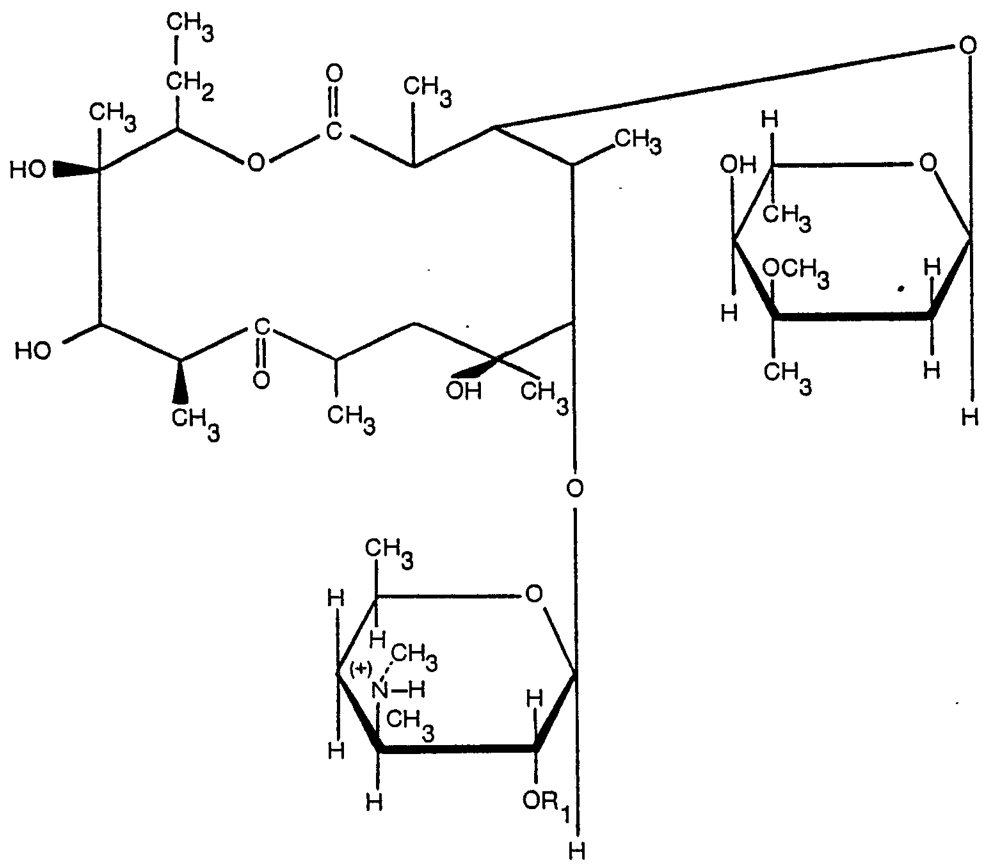

dans laquelle $R_1$ représente H ou $CH_3—CH_2—CO$.

2. Sel d'érythromycine avec l'acide mercaptosuccinique suivant la revendication 1.

3. Sel de l'ester propionique d'érythromycine avec l'acide mercapto-succinique suivant la revendication 1.

4. Sel de l'ester propionique d'érythromycine avec l'alpha-mercapto hémisuccinate de sodium suivant la revendication 1.

5. Sel d'érythromycine avec l'alpha-mercapto hémisuccinate de sodium suivant la revendication 1.

6. Procédé de préparation de dérivés thioliques d'érythromycine et de l'ester propionique d'érythromycine, dans lequel on fait réagir de l'érythromycine base ou son ester propionique avec de

l'acide mercapto-succinique ou le mercapto hémisuccinate de sodium, caractérisé en ce que la réaction est réalisée dans un solvant organique, à une température de 20 à 40°C et en présence d'eau.

7. Procédé suivant la revendication 6, caractérisé en ce que l'eau présente dans le solvant de réaction ne dépasse pas 20% en volume du solvant de réaction.

8. Procédé suivant la revendication 7, caractérisé en ce que le volume d'eau constitue de 4 à 5% de celui du solvant de la réaction.

9. Procédé suivant la revendication 6, caractérisé en ce que le solvant organique est l'acétone ou la méthylisobutylcétone.

10. Préparation pharmaceutique, caractérisée en ce qu'elle contient, comme ingrédient actif, un dérivé thiolique suivant la revendication 1, ainsi que des véhicules et des charges de type usuel et acceptables du point de vue pharmaceutique.

11. Préparation pharmaceutique ayant une activité antibactérienne et mucolytique, caractérisée en ce qu'elle contient, comme ingrédient actif, un dérivé suivant la revendication 1.

12. Préparation pharmaceutique suivant l'une ou l'autre des revendications 10 et 11, caractérisée en ce qu'elle est sous une forme permettant l'administration orale, en particulier sous la forme de capsules et de suspensions, et en ce qu'elle contient, comme ingrédient actif, un dérivé d'érythromycine suivant la revendication 1, ainsi que les excipients et véhicules usuels.

13. Préparation pharmaceutique suivant l'une ou l'autre des revendications 10 et 11, caractérisée en ce qu'elle est sous une forme permettant l'administration par la voie parentérale et par aérosol, et en ce qu'elle contient, comme ingrédient actif, un dérivé de l'ester propionique d'érythromycine suivant la revendication 1, ainsi que les excipients et véhicules usuels.

Fig.1

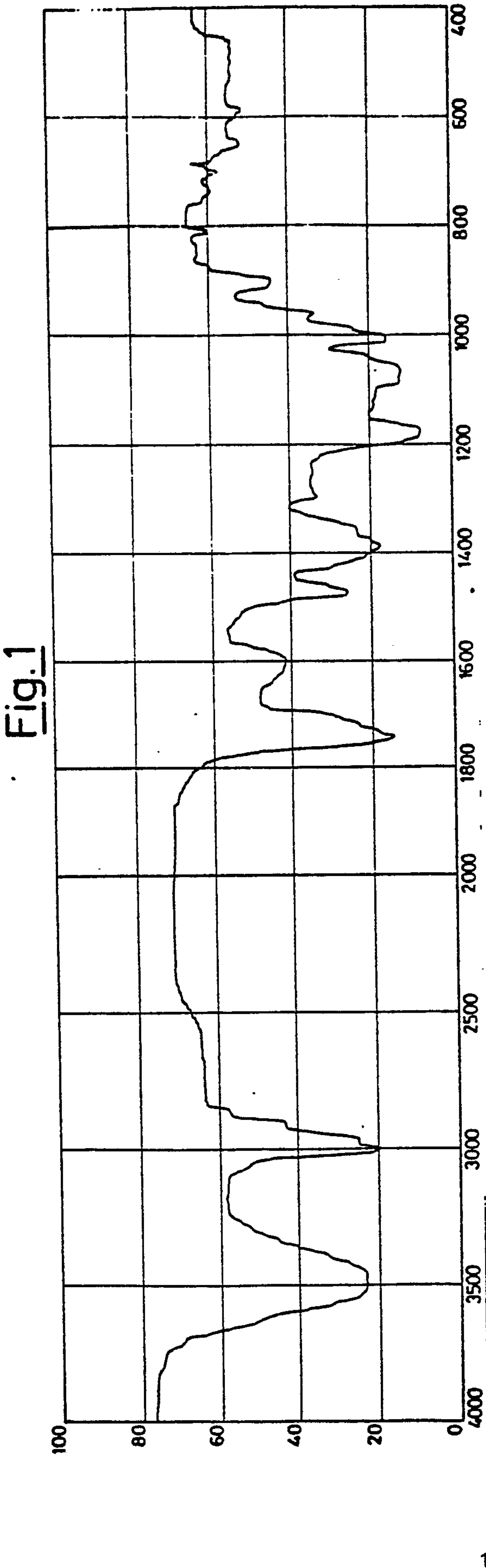
100
80
60
40
20
0
4000
3500
3000
2500
2000
1800
1600
1400
1200
1000
800
600
400

Fig.2

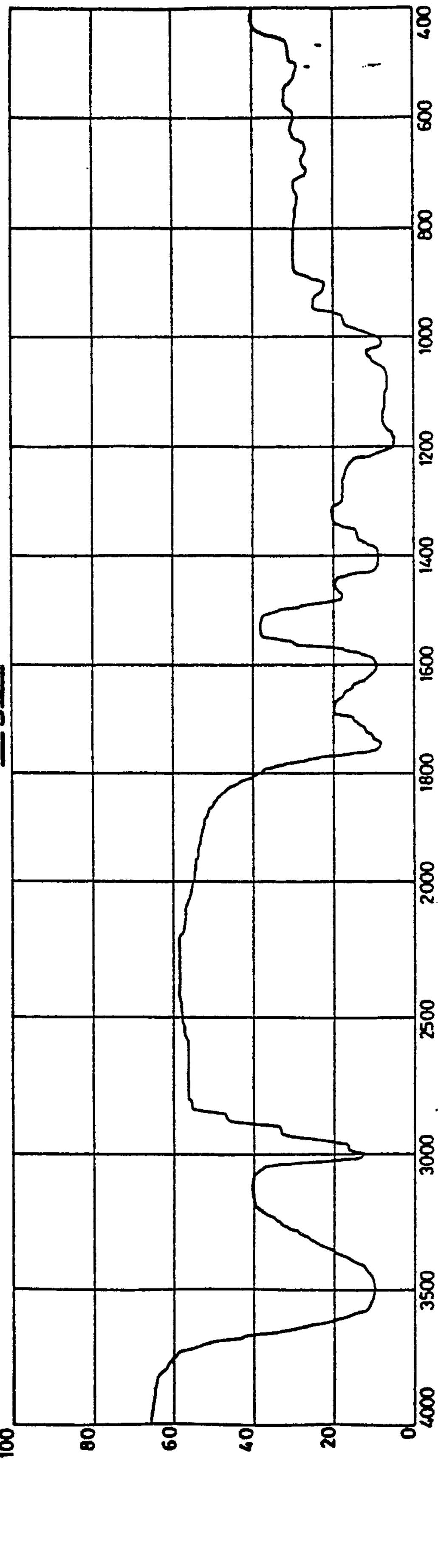
100
80
60
40
20
0
4000
3500
3000
2500
2000
1800
1600
1400
1200
1000
800
600
400